# EUROPÄISCHE PATENTSCHRIFT

(11) **EP 4 151 138 B1**
(45) Veröffentlichungstag und Bekanntmachung des Hinweises auf die Patenterteilung: **05.11.2025**
(21) Anmeldenummer: 22193396.3
(22) Anmeldetag: 01.09.2022
(51) Int. Cl.: A61B 1/00

(54) **AUSSENSCHAFT FÜR EIN ENDOSKOP UND ENDOSKOPIESYSTEM**
EXTERNAL SHAFT FOR AN ENDOSCOPE AND ENDOSCOPE SYSTEM
ARBRE EXTERNE POUR UN ENDOSCOPE ET SYSTÈME D'ENDOSCOPIE

(30) Priorität: 21.09.2021 DE 102021124453
(43) Veröffentlichungstag der Anmeldung: 22.03.2023
(73) Patentinhaber: Karl Storz SE & Co. KG, 78532 Tuttlingen (DE)
(72) Erfinder: HENI, Andreas, 78532 Tuttlingen (DE); HEDEMANN, Lars, 78532 Tuttlingen (DE)

(56) Entgegenhaltungen:
- US-A- 5 287 845
- US-A- 5 709 698
- US-A1- 2005 085 692
- US-A1- 2014 336 688

## Beschreibung

Die vorliegende Erfindung ist auf einen Außenschaft für ein Endoskop und auf ein Endoskopiesystem mit einem Außenschaft und einem Endoskop bezogen.

In EP 1 542 579 B1, EP 2 552 293 B1, WO 2018/165365 A2 und WO 2019/126683 A1 sind medizinische Instrumente mit dünnen langen Schäften beschrieben, die neben Einrichtungen zum Erfassen und Übertragen von Bildern auch einen oder mehrere Fluidkanäle zum Leiten von Spülfluid oder zum Absaugen eines Fluids von einem Situs aufweisen. Um eine vollständige Reinigung und Sterilisation zu vereinfachen oder zu gewährleisten, kann ein Außenschaft über den Schaft eines Endoskops gestülpt werden. Zwischen Außenschaft und Schaft des Endoskops verbleibt ein mantelförmiger Fluidkanal mit insbesondere im Wesentlichen kreisringförmigem Querschnitt, durch den ein Spülfluid zu dem distalen Ende geleitet oder ein Fluid von dem distalen Ende der Anordnung abgesaugt werden kann. Das Endoskop weist im Übergangsbereich zwischen dem proximalen Ende des Schafts und dem distalen Ende der Handhabungseinrichtung beispielsweise konvexe Bereiche auf, an denen das proximale Ende des Außenschafts befestigt werden kann. Verschiebbare, schwenkbare oder rotierbare Riegel am proximalen Ende des Außenschafts ermöglichen eine lösbare mechanische Verbindung.

US5287845 A beschreibt ein Endoskop für die transurethrale Chirurgie und weist einen Hauptkörper auf, der eine Optik und ein chirurgisches Instrument drehbar trägt. Ein Außenrohr des Endoskops ist an dem Hauptkörper befestigt und umschließt rohrförmig die Optik und das chirurgische Instrument, dadurch gekennzeichnet, dass das Außenrohr relativ zu den übrigen Endoskop teilen drehbar ist.

US5709698 A offenbart eine Rasierklingeneinheit mit einem stationären länglichen Außenrohr und einem drehbaren länglichen Innenrohr, wobei sowohl das Innen- als auch das Außenrohr an ihren proximalen Enden Naben zur Befestigung an einem Handstück aufweisen, das die innere Klinge relativ zur äußeren Klinge mit Kraft bewegt. Das äußere Rohr ist am proximalen Ende seiner rohrförmigen Oberfläche mit einer Flüssigkeitseinlassöffnung versehen, und ein Flüssigkeitsadapter kann selektiv am äußeren Rohr angebracht werden, um eine Möglichkeit zum Einführen von Spülflüssigkeit in die Flüssigkeitsöffnung zu schaffen. Der Adapter ist ein stückig mit Dichtungsflächen ausgebildet, die den Einsatz von O-Ringen und dergleichen überflüssig machen.

US2014/336688 offenbart eine Dilatationsvorrichtung, die ein Dilatationsinstrument, das einen distalen radial ausdehnbaren Abschnitt, der über einen länglichen Schaft mit einem Handhabeabschnitt gekoppelt ist, und einen aufweitbaren Überzug für den Ausdehnungsabschnitt des Dilatationsinstruments, der ein geschlossenes distales Ende und ein offenes proximales Ende hat, aufweist. Der Überzug weist einen Schaftabschnitt auf, der sich entlang des Schafts des Dilatationsinstruments erstreckt. Das offene proximale Ende des Überzugs ist lösbar mit dem distalen Ende des Handhabeabschnitts des Dilatationsinstruments gekoppelt. Ferner wird der Überzug an sich offenbart, der aus einem Netzmaterial besteht und einen Schaftabschnitt hat, der an dem offenen proximalen Ende mit einer Kopplungshülse verbunden ist.

Eine Aufgabe der vorliegenden Erfindung besteht darin, einen verbesserten Außenschaft für ein Endoskop und ein verbessertes Endoskopiesystem zu schaffen.

Diese Aufgabe wird gelöst durch die Gegenstände der unabhängigen Ansprüche.

Weitere Ausführungsformen sind in den abhängigen Ansprüchen definiert.

Ein Außenschaft zum Aufnehmen eines Schafts eines Endoskops umfasst einen proximalen Endbereich zum Aufnehmen eines proximalen Endbereichs des Schafts des Endoskops, eine Dichtfläche zum Anliegen an einer korrespondierenden Dichtfläche des Endoskops zum lokal fluiddichten Verschließen eines Zwischenraums zwischen Außenschaft und Schaft des Endoskops und eine nach radial innen ragende Knagge in dem proximalen Endbereich, zum proximalen Hintergreifen einer nach radial außen ragenden Knagge an dem proximalen Endbereich des Schafts des Endoskops, wobei die nach radial innen ragende Knagge an einem in axialer Richtung des Außenschafts elastisch verformbaren Abschnitt des proximalen Endbereichs des Außenschafts vorgesehen ist.

Der Außenschaft ist sowohl faktisch durch seine Länge, den Querschnitt seines Lumens und die Geometrie der Dichtfläche und der nach radial innen ragenden Knagge als auch rechtlich durch seine spezifische medizingeräterechtliche Zulassung für die ausschließliche Verwendung mit einem Endoskop einer vorbestimmten Bauart oder einer Bauart aus einer vorbestimmten Gruppe von Bauarten vorgesehen und ausgebildet. Dabei ist in der medizingeräterechtlichen Zulassung die Bauart oder die Gruppe von Bauarten eindeutig bezeichnet.

Der Außenschaft kann zur Bildung eines Endoskopiesystems aus dem Außenschaft und einem Endoskop für verschiedene medizinische Maßnahmen vorgesehen sein. Sowohl der Außenschaft als auch der Schaft des Endoskops können starr und gerade oder gekrümmt oder abschnittsweise oder vollständig flexibel ausgebildet sein.

Der Zwischenraum zwischen Außenschaft und Schaft des Endoskops weist insbesondere zumindest abschnittsweise einen ringförmigen, beispielsweise kreisringförmigen Querschnitt auf und erstreckt sich bis zu einem distalen Ende der Anordnung aus Außenschaft und Schaft des Endoskops. Der Zwischenraum kann sowohl zum Leiten eines Spülfluids zu diesem distalen Ende als auch zum Absaugen eines Fluids von dort vorgesehen und ausgebildet sein. Der Zwischenraum zwischen Außenschaft und Schaft des Endoskops kann in dem proximalen Endbereich einen erweiterten Querschnitt aufweisen und in einen beispielsweise radial angeordneten Fluidanschluss übergehen.

Die Dichtfläche an dem Außenschaft und die korrespondierende Dichtfläche des Endoskops verschließen, wenn sie aneinander anliegen, das proximale Ende des Zwischenraums zwischen Außenschaft und Schaft des Endoskops fluiddicht. Dazu ist die Dichtfläche des Außenschafts insbesondere konkav konisch und die Dichtfläche des Endoskops konvex konisch, wobei Durchmesser und Öffnungswinkel identisch oder ähnlich sind.

Die nach radial innen ragende Knagge in dem proximalen Endbereich des Außenschafts und die nach radial außen ragende Knagge an dem proximalen Endbereich des Schafts des Endoskops sind jeweils insbesondere als gerade oder gekrümmte Stege mit trapezförmigen oder rechteckigen Querschnitten ausgebildet. Während der vorgesehenen Verwendung des Außenschafts, bei der die Dichtfläche an dem Außenschaft gegen die Dichtfläche des Endoskops gepresst ist, ist die nach radial innen ragende Knagge in dem proximalen Endbereich des Außenschafts proximal der nach radial außen ragenden Knagge an dem proximalen Endbereich des Schafts des Endoskops angeordnet.

Während des Befestigens des Außenschafts an dem Endoskop wird der elastisch verformbare Abschnitt elastisch nach proximal verformt. Die resultierende elastische Rückstellkraft presst einen nach distal orientierten Oberflächenbereich der nach radial innen ragenden Knagge des Außenschafts gegen einen nach proximal orientierten Oberflächenbereich der nach radial außen ragenden Knagge an dem proximalen Endbereich des Schafts. Dadurch wird gleichzeitig die Dichtfläche an dem Außenschaft gegen die Dichtfläche des Endoskops gepresst.

Mit der nach radial innen ragenden Knagge an dem elastisch verformbaren Abschnitt des proximalen Endbereichs des Außenschafts weist der Außenschaft einen besonders einfachen mechanischen Aufbau auf. Insbesondere sind keine beweglichen Teile für die Verbindung von Außenschaft und Endoskop erforderlich. Dies kann eine kostengünstige Herstellung und eine große mechanische Robustheit ermöglichen. Insbesondere kann der Außenschaft oder zumindest der proximale Endbereich des Außenschafts einschließlich des elastisch verformbaren Abschnitts und der nach radial innen ragenden Knagge monolithisch herstellbar sein, beispielsweise als Gussteil, insbesondere als Spritzgussteil.

Bei einem Außenschaft, wie er hier beschrieben ist, weist der elastisch verformbare Abschnitt des proximalen Endbereichs insbesondere im Wesentlichen die Gestalt eines geraden oder gekrümmten Balkens auf, der an einem Ende oder an seinen beiden Enden mit dem übrigen proximalen Endbereich verbunden ist.

Der elastisch verformbare Abschnitt weist insbesondere die Gestalt eines Balkens auf, der parallel oder im Wesentlichen parallel zu einer Ebene orthogonal zu der Längsachse des Außenschafts ist. Beispielsweise ist der elastisch verformbare Abschnitt als in Richtung des Umfangs des proximalen Endbereichs angeordneter und der Kontur des proximalen Endbereichs folgender gekrümmter Balken ausgebildet.

Der elastisch verformbare Abschnitt wird beim Befestigen des Außenschafts an dem Endoskop insbesondere elastisch gebogen und/oder tordiert.

Bei einem Außenschaft, wie er hier beschrieben ist, sind insbesondere beide Enden des elastisch verformbaren Abschnitts mit dem übrigen proximalen Endbereich des Außenschafts verbunden, wobei der elastisch verformbare Abschnitt nahe seinen Enden eine erhöhte elastische Biegbarkeit aufweist.

Bei einem Außenschaft, wie er hier beschrieben ist, weist der elastisch verformbare Abschnitt insbesondere nahe seinen beiden Enden reduzierte Querschnitte auf.

Der elastisch verformbare Abschnitt wird insbesondere nahe seinen Enden in eine erste Richtung und zwischen seinen Enden in eine zweite, entgegengesetzte Richtung elastisch verformt.

Bei einem Außenschaft, wie er hier beschrieben ist, ist der elastisch verformbare Abschnitt insbesondere durch einen im Wesentlichen in umfänglicher Richtung des proximalen Endbereichs des Außenschafts verlaufenden Schlitz von dem übrigen proximalen Endbereich getrennt.

Der Schlitz kann eine konstante oder in seiner Längsrichtung variierende Breite aufweisen. Die Enden des Schlitzes definieren die Enden des elastisch verformbaren Abschnitts.

Anstelle eines Schlitzes kann eine Nut vorgesehen sein, die die Wandstärke des proximalen Endbereichs lokal wesentlich reduziert und damit die Elastizität des proximalen Endbereichs lokal vergrößert. Die Nut geht insbesondere von der Innenseite des proximalen Endbereichs aus.

Bei einem Außenschaft, wie er hier beschrieben ist, ist der proximale Endbereich des Außenschafts insbesondere im Wesentlichen trichterförmig oder becherförmig und nach proximal offen.

Der proximale Rand des proximalen Endbereichs des Außenschafts liegt insbesondere in einer Ebene orthogonal zu der Längsachse des Außenschafts. Die nach radial innen ragende Knagge ragt insbesondere in den von dem proximalen Endbereich trichterförmig oder becherförmig umschriebenen Hohlraum hinein. Die nach radial innen ragende Knagge ist insbesondere nahe dem proximalen Rand oder unmittelbar angrenzend an den proximalen Rand des proximalen Endbereichs des Außenschafts angeordnet.

Bei einem Außenschaft, wie er hier beschrieben ist, weist der proximale Endbereich insbesondere eine in axialer Richtung verlaufende und nach radial innen offene Nische zum Hindurchführen der nach radial außen ragenden Knagge an dem proximalen Endbereich des Schafts des Endoskops während des axialen Einführens des Schafts des Endoskops in den Außenschaft auf, wobei die nach radial innen ragende Knagge des Außenschafts ausgebildet und angeordnet ist, um während eines dem axialen Einführen nachfolgenden Rotierens des Außenschafts relativ zu dem Endoskop an der nach radial außen ragenden Knagge des Endoskops gleitend eine Position proximal der nach radial außen ragenden Knagge des Endoskops einzunehmen.

Die Nische ist insbesondere als in umfänglicher Richtung breite, in radialer Richtung flache und in axialer Richtung kurze Nut ausgebildet. Der Querschnitt der in axialer Richtung verlaufenden und nach radial innen offenen Nische ist insbesondere an den Querschnitt der nach radial außen ragenden Knagge des Endoskops angepasst, wobei die Querschnitte jeweils auf eine Schnittebene orthogonal zur Längsachse des Außenschafts und des Schafts des Endoskops bezogen sind. Bei dem dem axialen Einführen nachfolgenden Rotieren des Außenschafts relativ zu dem Endoskop wird der elastisch verformbare Abschnitt elastisch verformt.

Ein Außenschaft, wie er hier beschrieben ist, umfasst insbesondere ferner eine Rampenfläche an der Knagge des Außenschafts, zum Erzeugen einer den elastisch verformbaren Abschnitt verformenden Kraft in axialer Richtung des Außenschafts bei einer Rotation des Außenschafts relativ zu dem Endoskop.

Die Rampenfläche verläuft im Wesentlichen in umfänglicher Richtung, ist jedoch gegenüber einer Ebene orthogonal zur Längsachse des Außenschafts geneigt. Der Winkel zwischen der Rampenfläche und der Ebene orthogonal zu der Längsachse des Außenschafts ist von der Elastizität des elastisch verformbaren Abschnitts und der resultierenden Rückstellkraft und damit insbesondere von der Geometrie und dem Material des elastisch verformbaren Abschnitts abhängig. Der Winkel liegt insbesondere im Bereich von 5° bis 40° oder im Bereich von 10° bis 30°. Während des Rotierens des Außenschafts relativ zu dem Endoskop gleitet die nach radial außen ragende Knagge des Endoskops an der Rampenfläche der nach radial innen ragenden Knagge des Außenschafts.

Ein Außenschaft, wie er hier beschrieben ist, umfasst insbesondere ferner eine in die Rampenfläche übergehende Plateaufläche an der Knagge des Außenschafts.

Bei der vorgesehenen Verwendung liegt die nach radial außen ragende Knagge des Endoskops insbesondere an der Plateaufläche an der Knagge des Außenschafts an.

Bei einem Außenschaft, wie er hier beschrieben ist, drückt in einer Konfiguration, in der der Außenschaft in der vorgesehenen Weise mit dem Endoskop verbunden ist, die elastische Rückstellkraft des elastisch verformbaren Abschnitts die nach radial innen ragende Knagge des Außenschafts in axialer Richtung gegen die nach radial außen ragende Knagge des Endoskops und die Dichtfläche des Außenschafts gegen die korrespondierende Dichtfläche des Endoskops und arretiert damit den Außenschaft hinsichtlich einer Rotation relativ zu dem Endoskop reibschlüssig.

Der Reibschluss ist sowohl von den tribologischen Eigenschaften als auch von den geometrischen Eigenschaften, nämlich Größen und Neigungen der aneinander anliegenden Oberflächenbereiche abhängig. Reibschluss kann zwischen den beteiligten Oberflächenbereichen der nach radial innen ragenden Knagge des Außenschafts und der nach radial außen ragenden Knagge des Endoskops, und aufgrund der Konizität vor allem zwischen der Dichtfläche des Außenschafts und der korrespondierenden Dichtfläche des Endoskops entstehen.

Bei einem Außenschaft, wie er hier beschrieben ist, ist insbesondere keine Rastung oder andere teilweise oder vollständig formschlüssige Arretierung der rotatorischen Position des Außenschafts relativ zu dem Endoskop vorgesehen.

Vor allem der Reibschluss zwischen der konkav konischen Dichtfläche des Außenschafts und der konvex konischen korrespondierenden Dichtfläche des Endoskops kann stark genug sein, um eine Rastung oder eine andere teilweise oder vollständig formschlüssige Arretierung überflüssig zu machen.

Alternativ oder zusätzlich kann eine Rastung oder eine andere formschlüssige Verriegelung vorgesehen sein. Dies kann abhängig von den tribologischen Eigenschaften der verwendeten Werkstoffe und ihrer Oberflächen erforderlich oder vorteilhaft sein.

Bei einem Außenschaft, wie er hier beschrieben ist, ist insbesondere der Außenschaft einschließlich der nach radial innen ragenden Knagge aus Kunststoff gefertigt und zur einmaligen Verwendung vorgesehen und ausgebildet.

Der Außenschaft ist insbesondere nicht autoklavierbar und deshalb auch nicht oder nicht ohne Weiteres wiederverwendbar. Der Außenschaft ist jedoch insbesondere zur Verwendung mit einem Mehrweg-Endoskop, also einem Endoskop, das mehrfach verwendbar ist, vorgesehen und ausgebildet. Alternativ kann der Außenschaft vorgesehen und ausgebildet sein zur Verwendung mit einem Einweg-Endoskop, also einem Endoskop, das nach einer Verwendung zu entsorgen ist und nicht für eine Aufbereitung und Wiederverwendung vorgesehen ist,
Bei einem Außenschaft, wie er hier beschrieben ist, sind insbesondere an der Innenseite des proximalen Endbereichs mehrere nach radial innen ragende Knaggen zum Hintergreifen je einer korrespondierenden nach radial außen ragenden Knagge des Endoskops vorgesehen, wobei jede nach radial innen ragende Knagge an einem zugeordneten in axialer Richtung des Außenschafts elastisch verformbaren Abschnitt des proximalen Endbereichs vorgesehen ist.

Die mehreren nach radial innen ragenden Knaggen und die mehreren elastisch verformbaren Abschnitte sind insbesondere untereinander gleich und gleichmäßig über einen Umfang des proximalen Endbereichs verteilt. Insbesondere sind zwei nach radial innen ragende Knaggen an je einem elastisch verformbaren Abschnitt vorgesehen und einander gegenüberliegend angeordnet.

Ein Endoskopiesystem umfasst einen Außenschaft, wie er hier beschrieben ist, und ein Endoskop mit einem Schaft, einem proximalen Endbereich, einer zu der Dichtfläche des Außenschafts korrespondierenden Dichtfläche an dem proximalen Endbereich des Endoskops und einer nach radial außen ragenden Knagge zum Hintergreifen der nach radial innen ragenden Knagge an der Innenseite des proximalen Endbereichs des Außenschafts.

### Kurzbeschreibung der Figuren

Nachfolgend werden Ausführungsformen anhand der beigefügten Figuren näher erläutert. Es zeigen:
- Figur 1: eine schematische axonometrische Darstellung eines Endoskopiesystems;
- Figur 2: eine schematische axonometrische Darstellung eines Endoskops des Endoskopiesystems aus Figur 1;
- Figur 3: eine schematische axonometrische Darstellung eines proximalen Endes eines Außenschafts des Endoskopiesystems aus Figur 1;
- Figur 4: eine schematische Darstellung eines Schnitts durch das Endoskopiesystem aus den Figuren 1 bis 3;
- Figur 5: eine schematische Darstellung eines weiteren Schnitts durch das Endoskopiesystem aus den Figuren 1 bis 4;
- Figur 6: eine schematische Darstellung des proximalen Endbereichs des Außenschafts aus den Figuren 1 und 3 bis 5;
- Figur 7: eine weitere schematische Darstellung des proximalen Endbereichs des Außenschafts aus den Figuren 1 und 3 bis 6.

### Beschreibung der Ausführungsformen

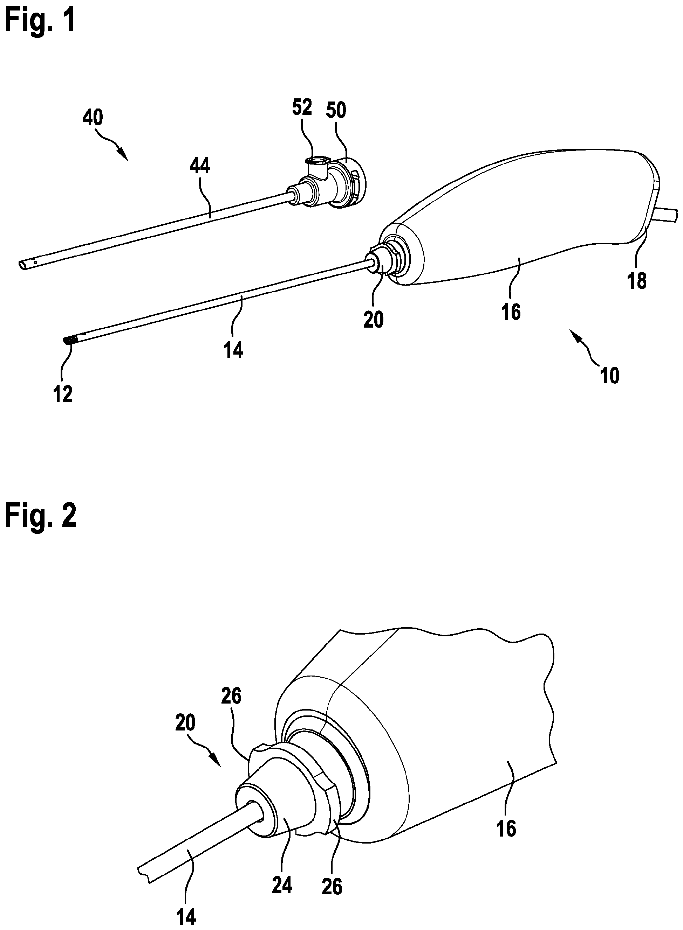

Figur 1 zeigt eine schematische axonometrische Darstellung eines Endoskopiesystems mit einem Endoskop 10 und einem Außenschaft 40. Das distale Ende 12 des Endoskops 10 wird durch einen Schaft 14 gebildet, der bei dem dargestellten Beispiel gerade und starr ausgebildet ist. Eine Handhabungseinrichtung 16 bildet das proximale Ende 18 des Endoskops 10. In einem proximalen Endbereich 20 des Schafts 14 geht dieser in die Handhabungseinrichtung 16 über.

Der Außenschaft 40 umfasst ein Schaftrohr 44 und einen proximalen Endbereich 50 mit einem Fluidanschluss 52, der bei dem dargestellten Beispiel als Spülanschluss ausgebildet ist.

Der Querschnitt des Lumens des Außenschafts, insbesondere von dessen Schaftrohr 44, ist so an den Querschnitt des Schafts 14 des Endoskops angepasst, dass der Schaft 14 des Endoskops 10 in den Außenschaft 40 und dessen Schaftrohr 44 vollständig eingeführt werden kann und dabei zwischen der äußeren Oberfläche des Schafts 14 des Endoskops 10 und der inneren Oberfläche des Außenschafts 40 ein Spalt verbleibt. Dieser Spalt weist insbesondere einen ringförmigen oder einen C-förmigen Querschnitt auf und erstreckt sich bis zu dem distalen Ende 12 des Endoskops 10. Durch den Spülanschluss 52 kann ein Spülfluid in den Außenschaft 40 eingeleitet werden, das durch den Spalt bis zu dem distalen Ende 12 des Endoskops 10 fließt und dort austritt.

Figur 2 zeigt eine vergrößerte schematische axonometrische Darstellung eines Teils des Endoskops 10 des Endoskopiesystems aus Figur 1, nämlich des proximalen Endbereichs 20 des Schafts 14 und des anschließenden distalen Endes der Handhabungseinrichtung 16. Im proximalen Endbereich 20 des Schafts 14 des Endoskops 10 sind ein Außenkonus 24 und proximal des Außenkonus 24 zwei Knaggen 26 vorgesehen. Die Knaggen 26 sind an zwei voneinander abgewandten Seiten des proximalen Endbereichs 20 vorgesehen. Jede Knagge 26 weist bei dem dargestellten Beispiel näherungsweise die Gestalt eines nach radial außen ragenden Stegs mit rechteckigem Querschnitt auf. Die äußeren Konturen der Knaggen 26 sind jeweils kreisbogenförmig und bilden Ausschnitte desselben Kreises, dessen Mittelpunkt auf der Längs- und Symmetrieachse des Schafts 14 liegt.

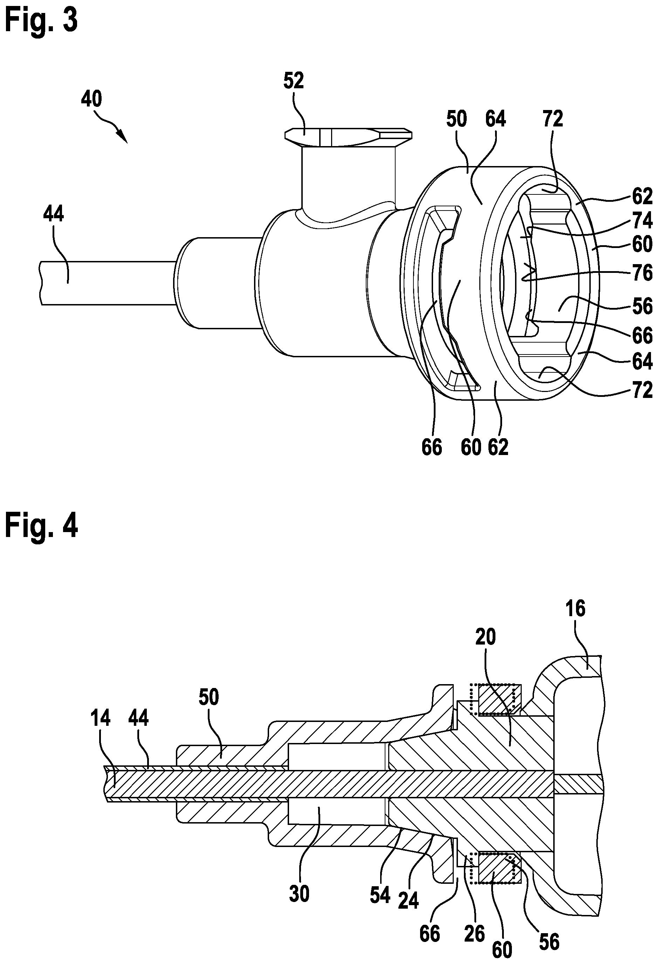

Figur 3 zeigt eine schematische und vergrößerte axonometrische Darstellung des proximalen Endbereichs 50 des Außenschafts des Endoskopiesystems aus Figur 1. Der proximale Endbereich 50 des Außenschafts 40 ist mit dem Schaftrohr 44 des Außenschafts 40 dauerhaft und starr mechanisch verbunden. Der Außenschaft 40 kann monolithisch gebildet sein, beispielsweise als ein Kunststoff-Spritzgussteil, das sowohl das Schaftrohr 44 als auch den proximalen Endbereich 50 umfasst. Alternativ kann das Schaftrohr 44 mit dem proximalen Endbereich 50 des Außenschafts 40 gefügt sein, beispielsweise durch eine Klebung oder eine Schweißung. In diesem Fall sind beispielsweise das Schaftrohr 44 aus Metall und der proximale Endbereich 50 des Außenschafts 40 aus Kunststoff gebildet.

Bei dem dargestellten Beispiel ist der Spülanschluss 52 radial angeordnet. Der Spülanschluss 52 kann als Luer-Kupplung ausgebildet sein.

Der proximale Endbereich 50 des Außenschafts 40 ist becherförmig ausgebildet und weist zwei einander gegenüberliegende, jeweils nach radial innen vorstehende Knaggen 56 auf. Bei dem dargestellten Beispiel sind die Knaggen 56 großflächig ausgebildet, die in Richtung parallel zur Längsachse des Außenschafts 40 gemessene Tiefe jeder Knagge 56 und die in Richtung des Umfangs gemessene Breite jeder Knagge 56 ist größer oder deutlich größer als die in radialer Richtung gemessene Höhe jeder Knagge 56. Jede Knagge 56 ist an einem elastisch verformbaren Abschnitt 60 des proximalen Endbereichs 50 des Außenschafts 40 angeordnet. Jeder elastisch verformbare Abschnitt 60 weist im Wesentlichen die Gestalt eines der äußeren Kontur des proximalen Endbereichs 50 folgend gekrümmten Balkens auf und ist über seine Enden 62, 64 mit dem übrigen proximalen Endbereich 50 monolithisch verbunden. Jeweils ein Schlitz 66 trennt jeden elastisch verformbaren Abschnitt 60 von dem übrigen proximalen Endbereich 50. Die Enden der Schlitze 66 definieren die Enden 62, 64 der elastisch verformbaren Abschnitte 60.

In umfänglicher Richtung zwischen den Knaggen 56 sind radiale Aussparungen oder Nischen 72 vorgesehen. Die Querschnitte der Nischen 72 sind an die Querschnitte der Knaggen 26 an dem proximalen Endbereich 20 des Endoskops 10 (vgl. Figuren 1, 2) angepasst, wobei die Querschnitte als Schnitte in Ebenen orthogonal zu der Längsachse des Außenschafts 40 angesehen werden. Deshalb können am Ende eines Einführens des Schafts 14 des Endoskops 10 (vgl. Figuren 1, 2) in den Außenschaft 40 die Knaggen 26 an dem proximalen Endbereich 20 des Endoskops 10 in axialer Richtung durch die Nischen 72 bewegt werden, bis sie in einer Ebene mit den Schlitzen 66 angeordnet sind.

Bei einer nachfolgenden Rotation des Außenschafts 40 relativ zu dem Endoskop 10 gleiten die Knaggen 26 an dem proximalen Endbereich 20 des Endoskops 10 (vgl. Figuren 1, 2) an Rampenflächen 74 an den Knaggen 56 und weiter an Plateauflächen 76 an den Knaggen 56 in dem proximalen Endbereich 50 des Außenschafts 40 entlang. Wenn die Knaggen 26 an dem proximalen Endbereich 20 des Endoskops 10 distal der Knaggen 56 in dem proximalen Endbereich 50 des Außenschafts 40 angeordnet sind, ist der Außenschaft 40 formschlüssig an dem Endoskop 10 gehalten. Bei dem Gleiten der Knaggen 26 an dem proximalen Endbereich 20 des Endoskops 10 entlang den Rampenflächen 74 an der Knaggen 56 werden die elastisch verformbaren Abschnitte 60 elastisch verformt, nämlich die Knaggen 56 nach proximal verschoben. Elastische Rückstellkräfte der elastisch verformbaren Abschnitte 60 drücken die Knaggen 56 von proximal gegen die Knaggen 26 an dem proximalen Endbereich 20 des Endoskops 10.

Figur 4 zeigt eine schematische Darstellung eines Schnitts durch einen Teil des Endoskopiesystems aus den Figuren 1 bis 3, nämlich durch den proximalen Endbereich 20 des Schafts 14 des Endoskops 10 und durch den proximalen Endbereich 50 des Außenschafts 40. Die Schnittebene der Figur 4 enthält die Längs- und Symmetrieachsen des Schafts 14 des Endoskops 10 und des Schaftrohrs 44 des Außenschafts 40 und ist orthogonal zu der Erstreckungsrichtung des Spülanschlusses 52. Die Schnittebene der Figur 4 schneidet die Knaggen 26 an dem proximalen Endbereich 20 des Schafts 14 des Endoskops 10 und die Knaggen 56 in dem proximalen Endbereich 50 des Außenschafts 40.

Der proximale Endbereich 50 des Außenschafts 40 weist einen zu dem Außenkonus 24 an dem proximalen Endbereich 20 des Schafts 14 des Endoskops 10 korrespondierenden Innenkonus 54 auf.

In Figur 4 ist die für die Verwendung des Endoskopiesystems vorgesehene Konfiguration gezeigt, in der die Knaggen 26 an dem proximalen Endbereich 20 des Schafts 14 des Endoskops 10 distal der Knaggen 56 in dem proximalen Endbereich 50 des Außenschafts 40 angeordnet sind. Die elastisch verformbaren Abschnitte 60 sind elastisch verformt, wobei sie von ihren in Figur 4 durch gestrichelte Linien angedeuteten Positionen in mechanisch spannungsfreiem Zustand nach proximal in die in Figur 4 in durchgezogenen Linien dargestellten Positionen verschoben sind. Die resultierende elastische Rückstellkraft drückt die Knaggen 56 in dem proximalen Endbereich 50 des Außenschafts 40 von distal gegen die Knaggen 26 an dem proximalen Endbereich 20 des Schafts 14 des Endoskops 10 und damit auch den Außenkonus 24 an dem proximalen Endbereich 20 des Schafts 14 gegen den Innenkonus 54 in dem proximalen Endbereich 50 des Außenschafts 40.

Durch die Neigung der Oberflächen von Außenkonus 24 und Innenkonus 54 ist die Flächennormalkraft zwischen denselben gegenüber der elastischen Rückstellkraft der elastisch verformten elastisch verformbaren Abschnitte 60 vergrößert. Die resultierende Reibung, insbesondere Haftreibung zwischen den Knaggen 26, 56 und vor allem zwischen Außenkonus 24 und Innenkonus 54 unterbindet eine unbeabsichtigte Rotation des Außenschafts 40 relativ zu dem Endoskop 10 zurück zu der Konfiguration, in der die Knaggen 26 an dem proximalen Endbereich 20 des Schafts 14 des Endoskops 10 in axialer Richtung durch die Nischen 72 (vgl. Figur 3) aus dem proximalen Endbereich 50 des Außenschafts 40 herausbewegt werden können.

In Figur 4 ist der Zwischenraum 30 zwischen der äußeren Oberfläche des Schafts 14 des Endoskops 10 und der inneren Oberfläche des Außenschafts 40 erkennbar. Der Zwischenraum 30 erstreckt sich mit ringförmigem Querschnitt bis zu dem distalen Ende 12 des Endoskops 10 (vgl. Figur 1). Das proximale Ende des Zwischenraums 30 ist in dem proximalen Endbereich 50 des Außenschafts 40 ringförmig erweitert. Der Außenkonus 24 an dem proximalen Endbereich 20 des Schafts 14 des Endoskops 10 und der Innenkonus 54 in dem proximalen Endbereich 50 des Außenschafts 40 bilden aneinander anliegende Dichtflächen, die den Zwischenraum 30 nach proximal fluiddicht abschließen.

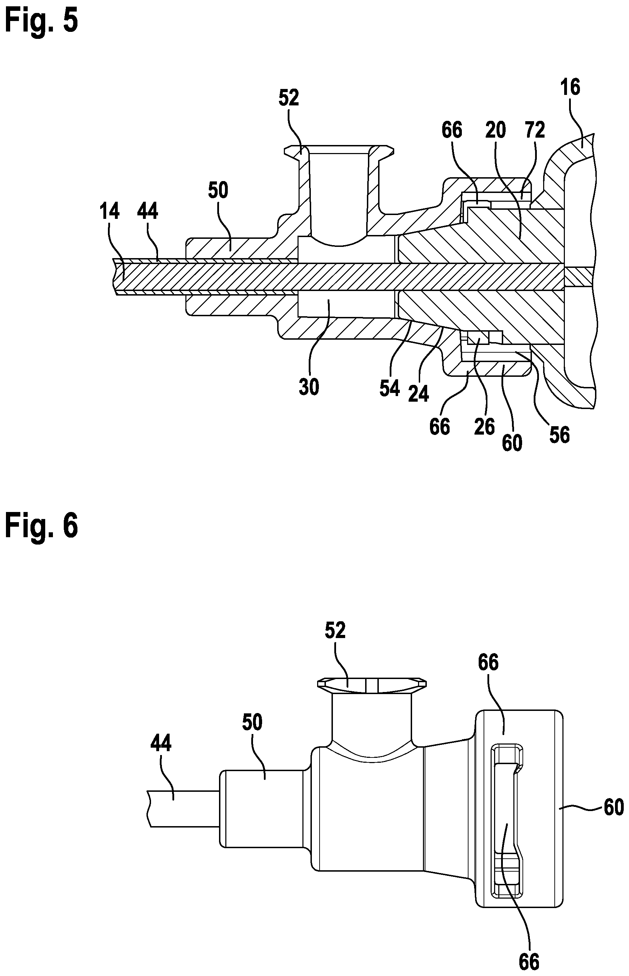

Figur 5 zeigt eine schematische Darstellung eines weiteren Schnitts durch einen Teil des Endoskopiesystems aus den Figuren 1 bis 4, nämlich den proximalen Endbereich 20 des Schafts 14 des Endoskops 10 und den proximalen Endbereich 50 des Außenschafts 40. Die Schnittebene der Figur 5 enthält die Längs- und Symmetrieachsen des Schafts 14 des Endoskops 10 und des Schaftrohrs 44 des Außenschafts 40, ist orthogonal zu der Schnittebene der Figur 4 und enthält die Richtung, in der sich der Spülanschluss 52 erstreckt. Die Schnittebene der Figur 5 schneidet die Nischen 72 in dem proximalen Endbereich 50 des Außenschafts 40.

Figur 6 zeigt eine weitere schematische Darstellung des proximalen Endbereichs 50 des Außenschafts 40. Die Zeichenebene der Figur 6 ist parallel zu der Schnittebene der Figur 5.

In Figur 6 sind die beiden Schlitze 66 teilweise in Deckung.

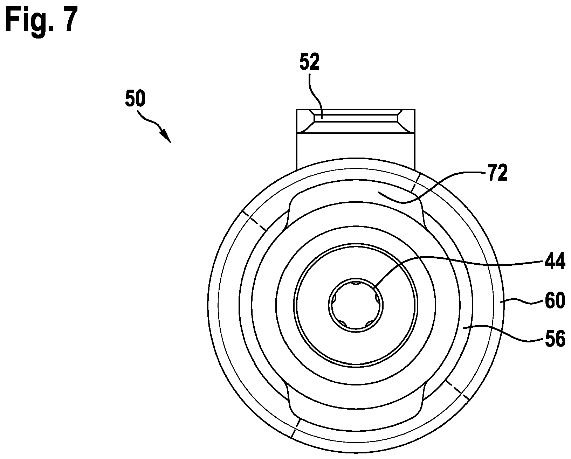

Figur 7 zeigt eine weitere schematische Darstellung des proximalen Endbereichs 50 des Außenschafts 40 aus den Figuren 1 und 3 bis 6. Die Schnittebene der Figur 7 ist orthogonal zu den Schnittebenen der Figuren 4 und 5 und zu der Zeichenebene der Figur 6 und orthogonal zu der Längs- und Symmetrieachse des Außenschafts 40.

Die an die Gestalt der Knaggen 26 an dem proximalen Endbereich des Schafts 14 des Endoskops 10 (vgl. Figuren 1, 2, 4, 5) angepassten Querschnitte der Nischen 72 und die zwischen den Nischen 72 nach innen ragenden Knaggen 56 sind erkennbar, die Schlitze 66 (vgl. Figuren 3, 4, 6) sind nicht sichtbar, ihre Enden sind jedoch durch gestrichelte Linien angedeutet.

### Bezugszeichen

- 10: Endoskop
- 12: distales Ende des Endoskops 10
- 14: Schaft des Endoskops 10
- 16: Handhabungseinrichtung des Endoskops 10
- 18: proximales Ende des Endoskops 10
- 20: proximaler Endbereich des Schafts 14 des Endoskops 10
- 24: Außenkonus an dem proximalen Endbereich 20 des Schafts 14
- 26: Knagge an dem proximalen Endbereich 20 des Schafts 14 des Endoskops 10
- 30: Zwischenraum zwischen dem Schaft 14 des Endoskops 10 und dem Außenschaft 40
- 40: Außenschaft für das Endoskop 10
- 44: Schaftrohr des Außenschafts 40
- 50: proximaler Endbereich des Außenschafts 40
- 52: Spülanschluss an dem proximalen Endbereich 50
- 54: Innenkonus in dem proximalen Endbereich 50 des Außenschafts 40
- 56: Knagge in dem proximalen Endbereich 50 des Außenschafts 40
- 60: elastisch verformbarer Abschnitt des proximalen Endbereichs 50 des Außenschafts 40
- 62: erste Ende des elastisch verformbaren Abschnitts 60
- 64: zweites Ende des elastisch verformbaren Abschnitts 60
- 66: Schlitz in dem proximalen Endbereich 50 des Außenschafts 40
- 72: Nische in dem proximalen Endbereich 50 des Außenschafts 40
- 74: Rampenfläche an der Knagge 56
- 76: Plateaufläche an der Knagge 56

## Patentansprüche

1. **Außenschaft** (40) zum Aufnehmen eines Schafts (14) eines Endoskops (10), mit:
einem **proximalen Endbereich** (50) zum Aufnehmen eines proximalen Endbereichs (20) des Schafts (14) des Endoskops (10);
einer **Dichtfläche** (54) zum Anliegen an einer korrespondierenden Dichtfläche (24) des Endoskops (10) zum lokal fluiddichten Verschließen eines Zwischenraums (30) zwischen Außenschaft (40) und Schaft (14) des Endoskops (10); **gekennzeichnet durch**:
eine **nach radial innen ragenden Knagge** (56) in dem proximalen Endbereich (50), zum proximalen Hintergreifen einer nach radial außen ragenden Knagge (26) an dem proximalen Endbereich (20) des Schafts (14) des Endoskops (10),
wobei der proximale Endbereich (50) einen in axialer Richtung des Außenschafts elastisch verformbaren Abschnitt (60) aufweist,
wobei die nach radial innen ragende Knagge (56) an dem **elastisch verformbaren Abschnitt** (60) des proximalen Endbereichs (50) des Außenschafts (40) vorgesehen ist,
sodass während des Befestigens des Außenschafts an dem Endoskop der elastisch verformbare Abschnitt elastisch nach proximal verformt wird und eine daraus resultierende Rückstellkraft einen nach distal orientierten Oberflächenbereich der nach radial innen ragenden Knagge gegen einen nach proximal orientierten Oberflächenbereich der nach radial außen ragenden Knagge gepresst wird und somit die Dichtfläche an dem Außenschaft gegen die Dichtfläche des Endoskops gepresst wird.

2. Außenschaft (40) gemäß dem vorangehenden Anspruch, bei dem
der elastisch verformbare Abschnitt (60) des proximalen Endbereichs (50) im Wesentlichen die Gestalt eines geraden oder gekrümmten **Balkens** aufweist, der an einem Ende (62) oder an seinen beiden Enden (62, 64) mit dem übrigen proximalen Endbereich (50) verbunden ist.

3. Außenschaft (40) gemäß dem vorangehenden Anspruch, bei dem **beide Enden** (62, 64) des elastisch verformbaren Abschnitts (60) mit dem übrigen proximalen Endbereich (50) des Außenschafts (40) verbunden sind,
der elastisch verformbare Abschnitt (60) nahe seinen Enden (62, 64) eine erhöhte **elastische Biegbarkeit** aufweist.

4. Außenschaft (40) gemäß einem der Ansprüche 2 und 3, bei dem
der elastisch verformbare Abschnitt (60) nahe seinen beiden Enden (62, 64) **reduzierte Querschnitte** aufweist.

5. Außenschaft (40) gemäß einem der vorangehenden Ansprüche, bei dem
der elastische verformbare Abschnitt (60) durch einen im Wesentlichen in umfänglicher Richtung des proximalen Endbereichs (50) des Außenschafts (40) verlaufenden **Schlitz** (66) von dem übrigen proximalen Endbereich (50) getrennt ist.

6. Außenschaft (40) gemäß einem der vorangehenden Ansprüche, bei dem
der proximale Endbereich (50) eine in axialer Richtung verlaufende und nach radial innen offene **Nische** (72) zum Hindurchführen der nach radial außen ragenden Knagge (26) an dem proximalen Endbereich (20) des Schafts (14) des Endoskops (10) während des axialen Einführens des Schafts (14) des Endoskops (10) in den Außenschaft (40) aufweist,
die nach radial innen ragende Knagge (56) des Außenschafts (40) ausgebildet und angeordnet ist, um während eines dem axialen Einführen nachfolgenden Rotierens des Außenschafts (40) relativ zu dem Endoskop (10) an der nach radial außen ragenden Knagge (26) des Endoskops (10) gleitend eine Position proximal der nach radial außen ragenden Knagge (26) des Endoskops (10) einzunehmen.

7. Außenschaft (40) gemäß einem der vorangehenden Ansprüche, ferner mit:
einer **Rampenfläche** (74) an der Knagge (56) des Außenschafts (40), zum Erzeugen einer den elastisch verformbaren Abschnitt (60) verformenden Kraft in axialer Richtung des Außenschafts (40) bei einer Rotation des Außenschafts (40) relativ zu dem Endoskop (10).

8. Außenschaft (40) gemäß einem der vorangehenden Ansprüche, bei dem in einer Konfiguration, in der der Außenschaft (40) in der vorgesehenen Weise mit dem Endoskop (10) verbunden ist, die **elastische Rückstellkraft** des elastisch verformbaren Abschnitts (60)
die nach radial innen ragende Knagge (56) des Außenschafts (40) in axialer Richtung gegen die nach radial außen ragenden Knagge (26) des Endoskops (10) drückt und
die Dichtfläche (54) des Außenschafts (40) gegen die korrespondierende Dichtfläche (24) des Endoskops (10) drückt und
damit den Außenschaft (40) hinsichtlich einer Rotation relativ zu dem Endoskop (10) **reibschlüssig arretiert.**

9. Außenschaft (40) gemäß einem der vorangehenden Ansprüche, bei dem
der Außenschaft (40) einschließlich der nach radial innen ragenden Knagge (56) aus **Kunststoff** gefertigt und zur **einmaligen Verwendung** vorgesehen und ausgebildet ist.

10. Außenschaft (40) gemäß einem der vorangehenden Ansprüche, wobei
an der Innenseite des proximalen Endbereichs (50) mehrere **nach radial innen ragende Knaggen** (56) zum Hintergreifen je einer korrespondierenden nach radial außen ragenden Knagge (26) des Endoskops (10) vorgesehen sind,
wobei jede nach radial innen ragende Knagge (56) an einem zugeordneten in axialer Richtung des Außenschafts (40) **elastisch verformbaren Abschnitt** (60) des proximalen Endbereichs (50) vorgesehen ist.

11. **Endoskopiesystem,** mit:
einem **Außenschaft** (40) gemäß einem der vorangehenden Ansprüche;
einem **Endoskop** (10) mit einem Schaft (14), einem proximalen Endbereich (20), einer zu der Dichtfläche (54) des Außenschafts (40) korrespondierenden Dichtfläche (24) an dem proximalen Endbereich (20), einer nach radial außen ragenden Knagge (26) zum Hintergreifen der nach radial innen ragenden Knagge (56) an der Innenseite des proximalen Endbereichs (50) des Außenschafts (40).

## Claims

1. An **outer shaft** (40) for receiving a shaft (14) of an endoscope (10), comprising:
a **proximal end region** (50) for receiving a proximal end region (20) of the shaft (14) of the endoscope (10);
a **sealing surface** (54) for abutting against a corresponding sealing surface (24) of the endoscope (10) for locally fluid-tight sealing of an intermediate space (30) between the outer shaft (40) and the shaft (14) of the endoscope (10);
**characterised by**:
a **radially inwardly projecting cleat** (56) in the proximal end region (50), for proximally engaging behind a radially outwardly projecting cleat (26) at the proximal end region (20) of the shaft (14) of the endoscope (10),
wherein the proximal end region (50) has a section (60) which is elastically deformable in the axial direction of the outer shaft,
wherein the radially inwardly projecting cleat (56) is provided on the **elastically deformable section** (60) of the proximal end region (50) of the outer shaft (40), such that during the fastening of the outer shaft to the endoscope, the elastically deformable section is elastically deformed proximally and a resulting restoring force presses a distally orientated surface region of the radially inwardly projecting cleat against a proximally orientated surface region of the radially outwardly projecting cleat and thus the sealing surface on the outer shaft is pressed against the sealing surface of the endoscope.

2. The outer shaft (40) according to the preceding claim, in which the elastically deformable section (60) of the proximal end region (50) is substantially in the form of a straight or curved **bar** connected at one end (62) or at both its ends (62, 64) to the remaining proximal end region (50).

3. The outer shaft (40) according to the preceding claim, in which **both ends** (62, 64) of the elastically deformable section (60) are connected to the remaining proximal end region (50) of the outer shaft (40), the elastically deformable section (60) has an increased **elastic bendability** near its ends (62, 64).

4. The outer shaft (40) according to one of claims 2 and 3, in which the elastically deformable section (60) has **reduced cross-sections** near both its ends (62, 64).

5. The outer shaft (40) according to one of the preceding claims, in which the elastically deformable section (60) is separated from the remaining proximal end region (50) by a **slot** (66) running substantially in the circumferential direction of the proximal end region (50) of the outer shaft (40).

6. The outer shaft (40) according to one of the preceding claims, in which the proximal end region (50) has a **recess** (72) running in the axial direction and open radially inwards for passing through the radially outwardly projecting cleat (26) on the proximal end region (20) of the shaft (14) of the endoscope (10) while the shaft (14) of the endoscope (10) is inserted axially into the outer shaft (40),
the radially inwardly projecting cleat (56) of the outer shaft (40) is formed and arranged to assume a position proximal to the radially outwardly projecting cleat (26) of the endoscope (10) in a sliding manner during a rotation of the outer shaft (40), which follows the axial insertion, relative to the endoscope (10) at the radially outwardly projecting cleat (26) of the endoscope (10).

7. The outer shaft (40) according to one of the preceding claims, further comprising:
a **ramp surface** (74) at the cleat (56) of the outer shaft (40), for generating a force deforming the elastically deformable section (60) in the axial direction of the outer shaft (40) upon rotation of the outer shaft (40) relative to the endoscope (10).

8. The outer shaft (40) according to one of the preceding claims, in which in a configuration in which the outer shaft (40) is connected to the endoscope (10) in the intended manner, the **elastic restoring force** of the elastically deformable section (60)
presses the radially inwardly projecting cleat (56) of the outer shaft (40) in the axial direction against the radially outwardly projecting cleat (26) of the endoscope (10), and
presses the sealing surface (54) of the outer shaft (40) against the corresponding sealing surface (24) of the endoscope (10) and
thereby **locks** the outer shaft (40) with respect to rotation relative to the endoscope (10) **in a frictionally-engaging manner.**

9. The outer shaft (40) according to one of the preceding claims, in which the outer shaft (40) including the radially inwardly projecting cleat (56) is made of **plastic** and is intended and designed for **single use.**

10. The outer shaft (40) according to one of the preceding claims, wherein
on the inner side of the proximal end region (50) is provided a plurality of **radially inwardly projecting cleats** (56) for engaging behind a corresponding radially outwardly projecting cleat (26) of the endoscope (10),
wherein each radially inwardly projecting cleat (56) is provided on an assigned **section** (60) of the proximal end region (50) that is **elastically deformable** in the axial direction of the outer shaft (40).

11. An **endoscope system,** comprising:
an **outer shaft** (40) according to one of the preceding claims;
an **endoscope** (10) with a shaft (14), a proximal end region (20), a sealing surface (24) on the proximal end region (20) corresponding to the sealing surface (54) of the outer shaft (40), a radially outwardly projecting cleat (26) for engaging behind the radially inwardly projecting cleat (56) on the inner side of the proximal end region (50) of the outer shaft (40).

## Revendications

1. **Tige externe** (40) pour la réception d'une tige (14) d'un endoscope (10), comportant :
une **zone d'extrémité proximale** (50) pour la réception d'une zone d'extrémité proximale (20) de la tige (14) de l'endoscope (10) ;
une **surface d'étanchéité** (54) pour l'application contre une surface d'étanchéité (24) correspondante de l'endoscope (10) pour la fermeture locale de manière étanche aux fluides d'un espace intermédiaire (30) entre la tige externe (40) et la tige (14) de l'endoscope (10) ; **caractérisée par** :
un **taquet** (56) faisant saillie radialement vers l'intérieur dans la zone d'extrémité proximale (50), pour venir en prise de manière proximale par l'arrière avec un taquet (26) faisant saillie radialement vers l'extérieur sur la zone d'extrémité proximale (20) de la tige (14) de l'endoscope (10),
dans laquelle la zone d'extrémité proximale (50) présente une section (60) élastiquement déformable dans la direction axiale de la tige externe,
dans laquelle le taquet (56) faisant saillie radialement vers l'intérieur est prévu sur la **section** (60) élastiquement déformable de la zone d'extrémité proximale (50) de la tige externe (40),
de sorte que, pendant la fixation de la tige externe à l'endoscope, la section élastiquement déformable est déformée élastiquement vers le côté proximal et une force de rappel qui en résulte presse une zone de surface orientée vers le côté distal du taquet faisant saillie radialement vers l'intérieur contre une zone de surface orientée vers le côté proximal du taquet faisant saillie radialement vers l'extérieur, et ainsi la surface d'étanchéité sur la tige externe est pressée contre la surface d'étanchéité de l'endoscope.

2. Tige externe (40) selon la revendication précédente, dans laquelle la section (60) élastiquement déformable de la zone d'extrémité proximale (50) présente sensiblement la forme d'une **poutre droite ou incurvée,** qui est reliée à une extrémité (62) ou à ses deux extrémités (62, 64) au reste de la zone d'extrémité proximale (50).

3. Tige externe (40) selon la revendication précédente, dans laquelle **les deux extrémités** (62, 64) de la section (60) élastiquement déformable sont reliées au reste de la zone d'extrémité proximale (50) de la tige externe (40), la section (60) élastiquement déformable présente une **flexibilité élastique accrue** à proximité de ses extrémités (62, 64).

4. Tige externe (40) selon l'une des revendications 2 et 3, dans laquelle la section (60) élastiquement déformable présente des **sections transversales réduites** à proximité de ses deux extrémités (62, 64).

5. Tige externe (40) selon l'une des revendications précédentes, dans laquelle la section (60) élastiquement déformable est séparée du reste de la zone d'extrémité proximale (50) par une **fente** (66) s'étendant sensiblement dans la direction circonférentielle de la zone d'extrémité proximale (50) de la tige externe (40).

6. Tige externe (40) selon l'une des revendications précédentes, dans laquelle la zone d'extrémité proximale (50) présente une **niche** (72) s'étendant dans la direction axiale et ouverte radialement vers l'intérieur pour le passage du taquet (26) faisant saillie radialement vers l'extérieur sur la zone d'extrémité proximale (20) de la tige (14) de l'endoscope (10) pendant l'insertion axiale de la tige (14) de l'endoscope (10) dans la tige externe (40),
le taquet (56) faisant saillie radialement vers l'intérieur de la tige externe (40) est réalisé et agencé pour prendre une position proximale du taquet (26) faisant saillie radialement vers l'extérieur de l'endoscope (10), en glissant sur le taquet (26) faisant saillie radialement vers l'extérieur de l'endoscope (10), pendant une rotation de la tige externe (40) par rapport à l'endoscope (10) suivant l'insertion axiale.

7. Tige externe (40) selon l'une des revendications précédentes, comportant en outre :
une surface de **rampe** (74) sur le taquet (56) de la tige externe (40), pour la génération d'une force déformant la section (60) élastiquement déformable dans la direction axiale de la tige externe (40) lors d'une rotation de la tige externe (40) par rapport à l'endoscope (10).

8. Tige externe (40) selon l'une des revendications précédentes, dans laquelle, dans une configuration dans laquelle la tige externe (40) est reliée à l'endoscope (10) de la manière prévue, la **force de rappel élastique** de la section (60) élastiquement déformable
presse le taquet (56) de la tige externe (40) faisant saillie radialement vers l'intérieur dans la direction axiale contre le taquet (26) de l'endoscope (10) faisant saillie radialement vers l'extérieur et
la surface d'étanchéité (54) de la tige externe (40) est pressée contre la surface d'étanchéité (24) correspondante de l'endoscope (10), et
bloque ainsi la tige externe (40) **par friction** par rapport à l'endoscope (10) en cas de rotation.

9. Tige externe (40) selon l'une des revendications précédentes, dans laquelle la tige externe (40), y compris le taquet (56) faisant saillie radialement vers l'intérieur, est fabriquée en **matière plastique** et est prévue et réalisée pour **une utilisation unique.**

10. Tige externe (40) selon l'une des revendications précédentes, dans laquelle
sur le côté intérieur de la zone d'extrémité proximale (50), il est prévu plusieurs **taquets** (56) faisant saillie radialement vers l'intérieur pour venir en prise par l'arrière respectivement un taquet (26) correspondant faisant saillie radialement vers l'extérieur de l'endoscope (10),
dans lequel chaque taquet (56) faisant saillie radialement vers l'intérieur est prévu sur une section (60) **élastiquement déformable** associée de la zone d'extrémité proximale (50) dans la direction axiale de la tige externe (40).

11. **Système d'endoscopie,** comportant :
une tige externe (40) selon l'une des revendications précédentes ;
un **endoscope** (10) comportant une tige (14), une zone d'extrémité proximale (20), une surface d'étanchéité (24) correspondant à la surface d'étanchéité (54) de la tige externe (40) sur la zone d'extrémité proximale (20), un taquet (26) faisant saillie radialement vers l'extérieur pour venir en prise par l'arrière avec le taquet (56) faisant saillie radialement vers l'intérieur sur le côté intérieur de la zone d'extrémité proximale (50) de la tige externe (40).
